Europäisches Patentamt

(19) European Patent Office

Office européen des brevets

(11) **EP 0 732 408 A2**

(12) **EUROPEAN PATENT APPLICATION**

(43) Date of publication:
18.09.1996 Bulletin 1996/38

(51) Int. Cl.⁶: **C12Q 1/68**, C07H 21/04

(21) Application number: 95203160.7

(22) Date of filing: 31.05.1990

(84) Designated Contracting States:
AT BE CH DE DK ES FR GB IT LI LU NL SE

(30) Priority: 31.05.1989 US 359293

(62) Application number of the earlier application in accordance with Art. 76 EPC: 90909245.4

(71) Applicant: AMOCO CORPORATION
Chicago Illinois 60680-0703 (US)

(72) Inventors:
• Shah, Jyotsna
Nashua, NH 03060 (US)
• Buharin, Amelia
St Paul, MN 55117 (US)
• Williams, Charlotte
Fairlee, VT 05045 (US)
• Mahan, Donald
Grafton, MA 01517 (US)
• Lane, David J.
Wheaton, IL 60187 (US)
• King, Walter
Wheaton, IL 60187 (US)

(74) Representative: Chapman, Paul William et al
Kilburn & Strode,
30 John Street
London WC1N 2DD (GB)

Remarks:
This application was filed on 17 - 11 - 1995 as a divisional application to the application mentioned under INID code 62.

(54) **Nucleic acid probes for the detection of chlamidia trachomatis**

(57) Nucleic acid probes capable of hybridizing to rRNA of Chlamydia trachomatis and not to rRNA of non-Chlamydia are described along with methods utilizing such probes for the detection of Chlamydia trachomatis in clinical samples.

EP 0 732 408 A2

## Description

This invention relates to detecting bacteria belonging to the species Chlamydia trachomatis and more specifically provides nucleic acid probes and compositions along with methods for their use for the specific detection of Chlamydia trachomatis.

The term "Chlamydia trachomatis" as used herein, refers to bacteria classified as such in Bergey's Manual of Systematic Bacteriology (P. H. A. Sneath, ed., 1986, PP. 729-736, Williams & Wilkins). Chlamydia trachomatis is a member of the genus Chlamydia, which has only one other member, Chlamydia psittaci. Chlamydia are a unique group of intracellular parasites. Chlamydia has a complex life cycle that is characterized by two forms, the elementary body (EB) which like a virus is infectious but not metabolically active, and the reticulate body (RB) which is metabolically active, not infectious and divides by binary fission like bacteria. The RB eventually matures into the EB which is released by the infected cell, a process which take about 2 days.

Because of their role in ocular, respiratory and sexually transmitted diseases, these organisms are a major health risk through the world (Nichols, R. L. and Manire, G. P.: Chlamydiae, in Microbiology 3rd Ed, Davis, B. D., Dulbecco, R., Eisen, H. N. and Ginsburg, H. S. {ed}. 1980, 776-784, Harper & Row). C. trachomatis is responsible for some 350 million cases of trachoma in the developing world (Dawson, C. R., Proceedings of the Fifth International Symposium on Human Chlamydial

Infections. Lund, Sweden, June 15-19, 1982, Mardh, P. A., Holmes, K. K., Oril, J. D., Plot, P., Schachter, J., {ed}, 1982, 71-81, Elsevier Biomedical Press, Amsterdam, New York, Oxford). This is particularly tragic since trachoma is also the leading cause of preventable blindness (Jones, B. R., 1975, Trans. Opthalmol. Soc. UK, 95: 16-33). Infection by this organism is now recognized as the most common sexually transmitted disease in the industrialized world (Centers for Disease Control, Division of sexually transmitted disease. Chlamydia trachomatis infections. Policy guidelines for prevention and control. MMWR 1985; 34: 53S-74S), accounting for more than 4 million cases in the United States alone (Judson FN. 1985, J.Reprod. Med. 30: 269-272). Direct and indirect costs run into billions of dollars.

Among the more serious complications of infection are ecotropic pregnancy and tubal infertility (Washington et al., 1987, JAMA 257: 2070-2072). These two manifestations of C. trachomatis infection reflect two further groupings: the trachoma biovar which canes ocular and genital infections and the lymphogranuloma veretrum (LGV) biovar. These two biovars are further subdivided in 15 serotypes: A, B, Ba, C (usually associated with follicular conjunctivitis), D through K, and L1 through L3.

Diagnosis of the disease is often lengthy and labor intensive. Cell culture is considered the "gold standard" for chlamydial diagnostics. Because of the time-consuming and tedious nature of cell culture, and the difficulty of growing many clinical isolates in tissue culture, a number of rapid laboratory methods have recently become available (see review by Barnes RC, 1989, Clin. Microbiol. Rev. 2: 119-136). The vast majority are antibody-based tests. While in certain instances these rapid assays have proven to be more sensitive or specific than cell culture, their utility lies in their rapidity.

It is an aspect of the present invention to provide nucleic acid probes which are specific for Chlamydia, which do not react with other bacteria or fungi which may be present in sampled materials, and which may be used in a variety of assay systems.

It is another aspect of the present invention to provide probes which can hybridize to target regions which can be rendered accessible to probes under normal assay conditions.

It is yet another aspect of the present invention to provide assays which avoid many of the disadvantages associated with traditional, multi-day culturing techniques.

While Kohne et al. (Biophysical Journal 8:1104-1118, 1968) discuss one method for preparing probes to rRNA sequences, they do not provide the teaching necessary to make Chlamydia trachomatis specific probes.

Pace and Campbell (Journal of Bacteriology 107:543-547, 1971) discuss the homology of ribosomal ribonucleic acids from diverse bacterial species and a hybridization method for quantitating such homology levels. Similarly, Sogin, Sogin and Woese (Journal of Molecular Evolution 1:173-184, 1972) discuss the theoretical and practical aspects of using primary structural characterization of different ribosomal RNA molecules for evaluating phylogenetic relationships. Fox, Pechman and Woese (International Journal of Systematic Bacteriology 27:44-57, 1977) discuss the comparative cataloging of 16S ribosomal RNAs as an approach to prokaryotic systematics. These references, however, fail to relieve the deficiency of Kohne's teaching with respect to Chlamydia trachomatis and in particular, do not provide Chlamydia trachomatis specific probes useful in assays for detecting Chlamydia trachomatis in clinical and other samples.

EP-A-0272009 does disclose probes which will hybridise to Chlamydia trachomatis. These probes are derived from selected regions of the 16S and 23S rRNA of Chlamydia trachomatis.

Ribosomes are of profound importance to all organisms because they serve as the only known means of translating genetic information into cellular proteins, the main structural and catalytic elements of life. A clear manifestation of this importance is the observation that all cells have ribosomes.

Ribosones contain three distinct RNA molecules which, at least in Escherichia coli, are referred to as 5S, 16S and 23S rRNAs. These names historically are related to the size of the RNA molecules, as determined by their sedimentation rate. In actuality, however, ribosomal RNA molecules vary substantially in size between organisms. Nonetheless, 5S, 16S, and 23S rRNA are commonly used as generic names for the homologous RNA molecules in any bacteria, and this convention will be continued herein.

As used herein, probe(s) refer to synthetic or biologically produced nucleic acids (DNA or RNA) which, by design or selection, contain specific nucleotide sequences that allow them to hybridize under defined predetermined stringencies, specifically (i.e., preferentially, see below - Hybridization) to target nucleic acid sequences. In addition to their hybridization properties, probes also may contain certain constituents that pertain to their proper or optimal functioning under particular assay conditions. For example, probes may be modified to carry detection ligands (e.g. fluorescien, 32-P, biotin, etc.), or to facilitate their capture onto a solid support (e. g., poly-deoxyadenosine "tails"). Such modifications are elaborations on the basic probe function which is its ability to usefully discriminate between target and non-target organisms in a hybridization assay.

Hybridization traditionally is understood as the process by which, under predetermined reaction conditions, two partially or completely complementary strands of nucleic acid are allowed to come together in an antiparallel fashion to form a double-stranded nucleic acid with specific and stable hydrogen bonds.

The stringency of a particular set of hybridization conditions is defined by the length and base composition of the probe/target duplex, as well as by the level and geometry of mispairing between the two nucleic acids.

Stringency may also be governed by such reaction parameters as the concentration and type of ionic species present in the hybridization solution, the types and concentrations of denaturing agents present, and/or the temperature of hybridization. Generally, as hybridization conditions become more stringent, longer probes are preferred if stable hybrids are to be formed. As a corollary, the stringency of the conditions under which a hybridization is to take place (e. g., based on the type of assay to be performed) will dictate certain characteristics of the preferred probes to be employed. Such relationships are well understood and can be readily manipulated by those skilled in the art.

As a general matter, dependent upon probe length, such persons understand stringent conditions to mean approximately 35°C-65°C in a salt solution of approximately 0.9 molar.

In accordance with the various principles and aspects of the present invention, there are provided nucleic acid probes and probe sets comprising DNA or RNA sequences which hybridize, under specific conditions, to the ribosomal RNA molecules (rRNA) or rRNA genes (rDNA) of Chlamydia trachomatis but which do not hybridize, under the same conditions, to the rRNA or rDNA of other related bacteria which may be present in test samples. Therefore the probe(s) of the present invention provide the basis for development of a valuable nucleic acid hybridization assay for the specific detection of Chlamydia trachomatis in clinical samples.

In our experience such nucleic acid hybridization based assays have been discovered to impart enhanced performance capabilities with respect to most currently available, microbiological methods for detection of bacteria in test samples, generally including:

a) increased sensitivity; i. e., the ability to detect fever said bacteria in a given sample;

b) potentially significant reductions in assay cost due to the use of inexpensive reagents and reduced labor;

c) accurate identification of even biochemically unusual strains of the target bacteria;

d) faster results because such tests do not require the isolation of the target bacterium from the sample prior to testing.

It has been discovered that other advantages incurred by directing the probes of the present invention against rRNA include the fact that the rRNAs detected constitute a significant component of cellular mass. Although estimates of cellular ribosome content vary, actively growing E. coli may contain upwards of 50,000 ribosomes per cell, and therefore 50,000 copies of each of the rRNAs (present in a 1:1:1 stoichiometry in ribosomes). Chlamydia trachomatis elementary bodies are known to contain far fewer ribosomes (hundreds). Nevertheless, this number is still larger than most other potential cellular target molecules such as genes or RNA transcripts thereof, which are less ideal since they are present in much lower abundance.

A further unexpected advantage is that the rRNAs (and the genes specifying them) appear not to be subject to lateral transfer between contemporary organisms. Thus, the rRNA primary structure provides an organism-specific molecular target, rather than a gene-specific target as would likely be the case, for example of a plasmid-borne gene or product thereof which may be subject to lateral transmission between contemporary organisms.

Additionally, the present invention provides probes to Chlamydia trachomatis rRNA target sequences which are sufficiently similar in all Chlamydia trachomatis strains tested that they can hybridize to the target region in all such Chlamydia trachomatis. Advantageously, these same rRNA target sequences are sufficiently different in most non-Chlamydia trachomatis rRNAs that, under conditions where probes 781, 860, 861, 879, 1153, 1203, 1220, 1318, 1319, 1320, 1321, 1322, 1323, 1325 and 1479 hybridize to Chlamydia trachomatis rRNAs and they do not hybridize to most non-Chlamydia trachomatis rRNAs. These probe characteristics are defined as inclusivity and exclusivity, respectively.

The other probes of the present invention, probes 783, 882, and 1324 are fully as inclusive for Chlamydia trachomatis strains as the probes mentioned above and, in addition, these probes also hybridize to a few other bacteria. Probe 782 is the only probe which hybridizes to Chlamydia psittaci and not to Chlamydia trachomatis.

The discovery that probes could be generated with the extraordinary inclusivity and exclusivity characteristics of those of the present invention with respect to Chlamydia trachomatis was unpredictable and unexpected.

Further understanding of the principles and aspects of the present invention may be ride by reference to the tables wherein:

Table 1 - Shows the nucleotide sequences of the preferred 16S rRNA-targeted probes of the present invention aligned upon their Chlamydial target nucleotide sequences. The corresponding portions of the 16S rRNA from E. coli is shown for comparison. RNA (target) sequences are written 5' to 3', probe sequences are DNA and written 3' to 5,. Probes are shown along with the "core" region of variation upon which their inclusivity and exclusivity behavior are based.

Table 2 - Shows the nucleotide sequences of the preferred 23S rRNA-targeted probes of the present invention aligned upon their Chlamydial target nucleotide sequences. The corresponding portions of the 23S rRNA from E. coli is shown for comparison. RNA (target) sequences are written 5' to 3', probe sequences are DNA and written 3' to 5'. Probes are shown along with the "core" region of variation upon which their inclusivity and exclusivity behavior are based.

Table 3 - Exemplifies the inclusivity behavior of the preferred 16S and 23S rRNA probes toward a representative sampling of Chlamydia trachomatis serovars in a liquid-hybridization assay.

Table 4 - Exemplifies the exclusivity behavior of the preferred 16S and 23S rRNA probes toward a representative sampling of non-Chlamydia trachomatis bacteria in a dot blot hybridization assay.

Probe Development Strategy.

The first step taken in the development of the probes of the present invention involved identification of regions of 16S and 23S rRNA which potentially could serve as target sites for Chlamydia trachomatis specific nucleic acid probes. As a practical matter, it is difficult to predict, a priori, which non-Chlamydia trachomatis organisms might be present in any test sample.

Because of the large number of such potential non-Chlamydia trachomatis bacteria, demonstrating exclusivity for any given probe sequence is not only unpredictable but also extremely difficult and laborious. A more rigorous criterion was adopted to obviate the need to know what non-Chlamydia trachomatis bacteria might be present in all test samples that ultimately will be screened using the probes.

This entailed knowledge of the phylogenetic relationships among Chlamydia trachomatis and between Chlamydia trachomatis and other groups of bacteria.

Specifically, an operating but previously unproven hypothesis was adopted that the exclusivity criterion could be satisfied by determining that if a particular target region in Chlamydia trachomatis rRNA could be identified which was sufficiently different from the homologous region in the rRNA of representative yet close evolutionary relatives of Chlamydia trachomatis, then a probe to such a sequence could be used to distinguish between Chlamydia trachomatis and the relatives by hybridization assay. Based on phylogenetic observations, it then was extrapolated that rRNA sequences of more distantly related organisms, even though their actual identity may not necessarily be known, should be predictably different in a particular region of sequence than the aforementioned close evolutionary relative of Chlamydia trachomatis. However, it cannot be predicted, a priori, whether such regions exist or if they do, where within the rRNA such regions will be located.

As the first step in identifying regions of Chlamydia trachomatis rRNA which could potentially serve as useful target sites for nucleic acid hybridization probes, complete nucleotide sequences of the 16S and 23S rRNAs from Chlanydia trachomatis was determined.

The nucleotide sequences were determined by standard laboratory protocols either by cloning (Maniatis et al., 1982, Molecular Cloning; A Laboratory Manual, Cold Spring Harbor Laboratory, New York, pp 545) and sequencing (Maxam and Gilbert, 1977, Proceedings of the National Academy of Science, USA 74:560-564: Sanger et al., 1977, Proceedings of the National Academy of Science, USA 74:5463-5467) the genes which specify the rRNAs, and/or by direct sequencing of the rRNAs themselves using reverse transcriptase (Lane et al., 1985, Proceedings of the National Academy of Science, USA 82:6955-6959).

The determined Chlamydia trachomatis rRNA nucleotide sequences were compared to other available rRNA nucleotide sequences, in particular to Chlamydia psittaci, which also was determined as part of this work.

Comparison of the sequences of Chlamydia trachomatis and its close relative Chlamydia psittaci proved especially valuable. Several regions of sequence were identified which appeared to be different in the two species of Chlamydia

and between <u>Chlamydia</u> <u>trachomatis</u> and non-<u>Chlamydia</u> bacteria. The locations of these regions within the 16S and 23S rRNA sequences are shown in Tables 1 and 2, respectively.

Oligonucleotide probes, 28 - 36 nucleotides in length, were designed which would hybridize preferentially to <u>Chlamydia</u> <u>trachomatis</u>, <u>Chlamydia</u> <u>psittaci</u> or both. These were designed 1) to maximally utilize the nucleotide sequence differences useful for distinguishing <u>Chlamydia</u> <u>trachomatis</u> from <u>Chlamydia</u> <u>psittaci</u> or <u>Chlamydia</u> from other bacteria (indicated as upper case letters in the region of Core Variation, Tables 1 and 2) and, 2) to minimize the effect of self complementarity both locally within the target rRNA and between probe molecules. Optimizing these parameters as well as others discussed above (Background) results in probes of preferred specificity and hybridization efficiency.

Table 3 exemplifies the inclusivity behavior of the preferred probes toward a representative sampling of <u>Chlamydia</u> <u>trachomatis</u> and a few non-<u>Chlamydia</u> <u>trachomatis</u> bacteria in a liquid hybridization assay.

Table 4 exemplifies the exclusivity behavior of the preferred probes toward a representative sampling of non-<u>Chlamydia</u> <u>trachomatis</u> bacteria in a dot-blot hybridization assay.

<u>Physical Description of the Probes</u>.

The foregoing probe selection strategy yielded a number of probes useful for identifying <u>Chlamydia</u> <u>trachomatis</u> bacteria in samples. Probes 781, 782, 783, 860, 861, 879, 882, 1153 and 1203 are designed from 16S rRNA sequences and probes 1318 through 1325 and probe 1479 are designed from 23S rRNA sequences. The following preferred oligonucleotide probes are disclosed herein:

16S rRNA-Targeted Probes:

```
Probe  781: 5'-CTTTAACGTTACTCGGATGCCCAAATATCGCCACAT-3'

Probe  782: 5'-CTTTAATATGTTTTAGATGCCTAAACATACCACAT-3'

Probe  783: 5'-CGGAAAACGACATTTCTGCCGCGGTCAAATACATG-3'

Probe  860: 5'-CGCTCAAATCCAGCGGGTATTAACCGCCTTCTC-3'

Probe  861: 5'-GCCGACTCGGGGTTGAGCCCCGATCTTTGACAA-3'

Probe  879: 5'-CGGATGGGGTTGAGACCATCCACATCAA-3'

Probe  882: 5'-TGTGTATATGTCCTTGCGGAAAACGACATTTCTGC-3'

Probe 1153: 5'-CCACTAAACAATCGTCGAAACAATTGCTCCGTTCG-3'

Probe 1203: 5'-CCACTAAACAATTGCCGAAACAATTGCTCCGTTCG-3'
```

23S rRNA-Targeted Probes:

```
Probe 1220: 5'-CCGGGGCTCCTATCGTTCCATAGTCACCCTAAAAG-3'
```

```
Probe 1318: 5'-TACCTCCGGGTCTTTGCTTATCACCAGCTCGCC-3'

Probe 1319: 5'-GTATTCAGCATGCAATGGTAGTCTATTACTCTA-3'

Probe 1320: 5'-TCGGCAGGTGTCGCTTTGCATACCTATGTATTC-3'

Probe 1321: 5'-CGAGCCTTATCAGCTCGGTTTAGGCTATTCCCC-3'

Probe 1322: 5'-AACTAGGAGTCCTGATCCTTTATCCTCAATCCT-3'

Probe 1323: 5'-TCAGGTGTTGAGGTCGGTCTTTCTCTCCTTTCG-3'

Probe 1324: 5'-AGATTCCCCTTGATCGCGACCTGATCTTATGTT-3'

Probe 1325: 5'-AACCGTTCTCATCGCTCTACGGACTCTTCCAAT-3'

Probe 1479: 5'-CTCCTACCGCGTGTCTTATCGACACACCCGCGA-3'
```

The probes and their target sequences in the 16S and 23S rRNAs of Chlamydia trachomatis and Chlamydia psittaci are shown in Tables 1 and 2. The corresponding nucleotide positions of the E. coil 16S and 23S rRNAs also are shown. Since the E. coil sequences were among the first full 16S and 23S sequences obtained, the assigned position numbers are a convenient standard for explicitly identifying the homologous regions in the Chlamydia rRNAs under consideration.

More than one probe has been designed to a number of the target regions shown in Tables 1 and 2 corresponding variously to complements of the sequences of Chlamydia trachomatis serovars LGV or K, or Chlamydia psittaci, or all three. The particular sequence upon which each probe is based (i.e., is complementary to) is provided in Tables 1 and 2 by inspection of the aligned probe and target sequences. Thus, for example, it can be seen in Table 1 that probe 1203 is complementary to the Chlamydia trachomatis serovar LGV sequence through this target region and the related probe 1153 is complementary to the C. trachomatis serovar K sequence. However, it is expected (and desirable) that some cross-hybridization between serovars by one or both probes will take place.

Likewise, probes 781 and 782 are based on Chlamydia trachomatis (LGV is identical to K in this region) and Chlamydia psittaci 16S rRNA sequences, respectively.

The specific hybridization behaviors of the probes described above are dependent to a significant extent on the assay format in which they are employed. Conversely, the assay format will dictate certain of the optimal design features of particular probes. The "essence" of the probes of the invention is not to be construed as restricted to the specific string of nucleotides in the probes named above. For example, the length of these particular oligonucleotides was optimized for use in the dot blot assay (and certain other anticipated assays) described below. It is well known to one skilled in the art that optimal probe length will be a function of the stringency of the hybridization conditions chosen and hence the length of the instant probes may be altered in accordance therewith. Also, in considering sets comprised of more than one probe, it is desirable that all probes behave in a compatible manner in any particular format in which they are both employed. Thus, the exact length of a particular probe will to a certain extent reflect its specific intended use.

The "essence" of the probes described herein resides in the discovery and utilization of the Chlamydia trachomatis specific sequences described above and given in Tables 1 and 2 (Core Variation).

Hybridization Analysis of Probe Behavior.

The sequence data in Tables 1 and 2 suggest that the probes of the present invention should exhibit a variety of useful hybridization properties with respect to the specific detection of Chlamydia trachomatis, Chlamydia psittaci or both to the exclusion of other bacteria. However, relatively few Chlamydia trachomatis and Chlamydia psittaci sequences were inspected. It is possible that sequence variation might exist in other Chlamydia trachomatis serotypes not inspected by sequence analysis. Such variation might reduce or eliminate hybridization by the prospective probes to some or many untested Chlamydia trachomatis serotypes.

Equally as important as the inclusivity behavior of the probes, is their exclusivity behavior, i.e., their reactivity toward non-Chlamydia bacteria. The number and types of non-Chlamydia strains which might be encountered in a potentially Chlamydia containing test sample are extremely large.

Therefore, the behavior of the probes toward representative Chlamydia trachomatis and non-Chlamydia trachomatis bacteria was determined by hybridization analysis using a liquid hybridization and a dot blot procedures.

While hybridization data for each of the individual probes of the present invention are provided, it should be noted that useful combinations (sets) of probes which exhibit hybridization behaviors that are the sum of the individual probes also is explicitly predicted by the data.

Example 1: Inclusivity Analysis of probe hybridization behaviour in liquid-hybridization assay.

The probes of the present invention or derivatives thereof could potentially be of significant value in a variety of hybridization formats. One such format, a dual probe, sandwich-type hybridization assay formats (e.g. the homopolymer capture, dual probe, liquid hybridization format described in USSN 277, 579; USSN 169,646, or USSN 233,683 equivalent to US-A-5147778, US-A-5370992 and US-A-5084565, respectively), is used in this example. In general in such an application, an oligonucleotide probe is modified at its 3' terminus to contain a tract or deoxyadenosine (dA) residues ca. 20 - 200 residues long. This would be used to "capture" the target rRNA (following liquid hybridization) from the test sample onto a solid support (e.g., beads, plastic surface, filter, etc.) which had been suitably derivatized with poly-deoxythymidine (dT) for this purpose. A second probe is used as a "detection" probe and would be derivatized by some detectable ligand (e.g. 32-P, fluorescien, biotin, etc.). In principle, the detection probe could be an oligonucleotide or a longer DNA or RNA probe. Detection of the presence of the target nucleic acid in a test sample then is indicated by the capture of the detection ligand onto the solid surface through the series of hybridization interactions:

```
SOLID   |            \       (TARGET NUCLEIC ACID)            /
        |TTTTTTTTn    _____/
SUPPORT |    ||||||      |||||||||||||    |||||||||||||||
           nAAAAAAAAA(Capture Probe)   (Detection Probe)
                                        |              |
                                      Ligand        Ligand
```

This could occur only if the target nucleic acid is present in the test sample.

In this example, each oligonucleotide individually is used as a capture probe (for this purpose ca. 200-500 dA residues were appended to their 3' termini using terminal deoxynucleotidyl transferase). P-32 labeled "riboprobes," specific for either 16S or 23S rRNA, were prepared as described below and used as "generic" detection probes.

Both riboprobes were prepared by transcription from plasmid vectors containing portions of the E. coli (16S rRNA) or C. psittaci (23S rRNA) genes. The 16S riboprobe is an antisense transcript, ca. 567 nucleotides long, generated from a HindIII subfragment of the E. coli 16S rRNA gene (E. coli positions 1 to 567). The 23S riboprobe is an antisense transcript, ca. 855 nucleotides long, generated from a PstI-EcoRI portion of the C. psittaci 23S rRNA gene (E. coli positions 1 to 855). Radioactive phosphorous 32 is incorporated into the riboprobes during the transcription reactions by using P-32 substituted nucleotide triphosphate precursors according to standard protocols.

Detection of the presence of the target nucleic acid in the test sample then is indicated by the capture of the detection ligand (P-32) onto the solid surface through series of hybridization interactions described above.

The inclusivity behaviors of the oligonucleotide probes were tested in a version of the liquid hybridization format discussed above.

The 16S and 23S riboprobes are expected to hybridize to all Chlamydia and non-Chlamydia 16S and 23S rRNAs, respectively. Therefore, a positive hybridization signal in this example assay in indicative of the hybridization behavior of the oligonucleotide probe employed in each experiment. Alternatively, these oligonucleotide probes could have been used to capture and detect P-32-labeled Chlamydia 16S and 23S rRNAs prepared, for example, by growth of the Chlamydia strains in radioactive phosphorous.

Fifteen serotypes of Chlamydia trachomatis have been tested. Serovar K was obtained from the American Type Culture Collection (ATCC). The rest (serovars A-LGV), were obtained from the Washington Research Foundation.

Briefly, formalin-fixed Elementary Bodies (EBs) of the indicated serovars of <u>Chlamydia</u> <u>trachomatis</u> were lysed in a solution of 1.0 mg/ml proteinase-K (Boehranger Mannheim Corp.) and 1.6% Sarkosine (Sigma) at 65°C for 15 minutes (final volume of 0.07ml). The samples were removed from the incubator and an equal volume of 5.0 M GuSCN (in 0.1M Tris-HCl pH 7.0, 10% dextran sulphate) containing poly-dA tailed capture probe and P-32 labeled detector probe (either 16S or 23S riboprobes appropriate to each oligonucleotide probe being tested) at a concentration of 80 ng/ml and 30 ng/ml (specific activity $1 \times 10^9$ cpm/ug) was added.

Hybridization of the probes to the target nucleic acids was allowed to proceed at 37°C for 30 minutes after which time the target complex (dA capture probe : target : detector probe ) was captured onto oligo-dT derivatized magnetic beads. This capture was accomplished by adding one tenth of a milliliter (0.1 ml) of a mixture containing 0.0625% (w/v) magnetic beads, 4% BSA, 10 mN EDTA, 0.2% Sarkosine, 0.1M Tris-HCl pH 8.0, and 0.05% Bronopol to the hybridization reaction and incubating the mixture for 5 minutes at 37°C.

Following the capture of the probe/target complexes onto the beads, 0.2 ml of a solution containing 1.0M GuSCN, 10mM EDTA, 0.1M Tris pH 7.0, 0.5% Sarkosine, 0.2% BSA, and 0.1% antifoam was added to the reaction tube, and the tube placed in a magnetic field. The beads (with the attached probe/target complexes) were drawn to the sides of the tube by the magnetic field and the liquid phase (containing the non-hybridized target and detector molecules) was removed from the tube by aspiration.

The captured target complex remaining in the tube was washed in this manner 2 more times.

The separated beads finally were suspended in 0.1 ml of a solution containing 3.25M GuSCN, 65mM EDTA, 0.1M Tris-HCl, pH 7.0, 0.5% Sarkosine and 0.5% BSA and incubated at 37°C for 5 minutes. This treatment releases the target complex from the bead via the "melting" of the dT:dA bond between the bead and the dA-tailed capture probe.

The solution was removed and mixed with a fresh aliquot of beads (0.1 ml) and the target complex was "recaptured" at 37°C for 5 minutes as described above. The wash procedure described above also was repeated.

After the third wash and separation, the beads were resuspended in 0.1 ml of the wash buffer and the beads were filtered onto a nitrocellulose membrane and exposed to x-ray film.

The extent of hybridization of each probe was determined by visual inspection of the exposed x-ray film. The results are shown in Table 3. ++++ indicates a hybridization signal equivalent to that of the "control" <u>Chlamydia</u> <u>trachomatis</u> serovar for which a perfect match between probe and target sequences has been explicitly determined by sequence analysis. +++, ++, + and - represent, respectively, very strong, strong, weak and undetectable hybridization signals.

Hybridization of the probes to approximately $1 \times 10^6$ of the non-<u>Chlamydia</u> (negative control) bacteria shown in Table 3 was scored in the same fashion.

Example 2: Dot blot analysis of probe hybridization behavior.

Dot blot analysis, in accordance with well known procedures, involves immobilizing a nucleic acid or a population of nucleic acids on a filter such as nitrocellulose, nylon, or other derivatized membranes which readily can be obtained commercially, specifically for this purpose. Either DNA or RNA can be easily immobilized on such a filter and subsequently can be probed or tested for hybridization under any of a variety of conditions (i.e., stringencies) with nucleotide sequences or probes of interest. Under stringent conditions, probes whose nucleotide sequences have greater complementarity to the target sequence will exhibit a higher level of hybridization than probes containing less complementarity.

For the experiment shown in Table 4, one tenth of a microgram of purified RNA (Lane <u>et al</u>., 1985, Proceedings of the National Academy of Science, USA 82:6955-6959) from each of the indicated organisms was spotted on nitrocellulose filters. The oligo- nucleotide probes were end-labeled with radioactive phosphorous 32, using standard procedures.

For the oligonucleotide probes described herein, hybridization to rRNA targets at 60°C for 14-16 hours (in a hybridization solution containing 0.9 M NaCl, 0.12 M Tris-HCl, pH 7.8, 6 mM EDTA, 0.1 M KPO4, 0.1% SDS, 0.1% pyrophosphate, 0.002% ficoll, 0.02% BSA, and 0.002% polyvinylpyrrolidine), followed by three 15 minute post-hybridization washes at 60°C (in 0.03 M NaCl, 0.004 M Tris-HCl, pH 7.8, 0.2 mM EDTA, and 0.1% SDS) to remove unbound probes, would be sufficiently stringent to produce the levels of specificity demonstrated in Table 4.

Following hybridization and washing as described above, the hybridization filters were exposed to X-ray film and the intensity of the signal "scored" visually with respect to control spots of known amount of target material (RNA) as described above (Example 1).

While the description of the invention has been made with reference to detecting rRNA, it will be readily understood that the probes described herein and probes complementary to those described herein also will be useful for the detection of the genes (DNA) which specify the rRNA and, accordingly, such probes are to be deemed equivalents to the described probes and encompassed within the spirit and scope to the present invention and the appended claims.

TABLE 1: CHLAMYDIA 16S rRNA PROBES AND TARGET SEQUENCES

```
Pos. # (E. coli)      63                                                    105
                       |                                                     |
Escherichia coli     GUCGAACGGUAACAGGAAGAAGCUUGCUUCUUUGCUGACGAGUGGCG
Chlamydia psittaci   GUCGAACGGAAUAAUGACUU--------CGGUUGUUAUUUAGUGGCG
C. trachomatis LGV   GUCGAACGGAGCAAUUGUUU--------CGGCAAUUGUUUAGUGGCG
C. trachomatis K     GUCGAACGGAGCAAUUGUUU--------CGACGAUUGUUUAGUGGCG
Core Variation                 GUuu--------cgRCRAuuG                 .
Probe 1203             GCTTGCCTCGTTAACAAA--------GCCGTTAACAAATCACC-5'
Probe 1153             GCTTGCCTCGTTAACAAA--------GCTGCTAACAAATCACC-5'


Pos. # (E. coli)      179                               198
                       |                                 |
Escherichia coli     GCAUAACGU-CGC-----------AAGACC---AAAGAG
Chlamydia psittaci   GAAUGUGGU-AUGUUUAGGCAUCUAAAACAUAUUAAAGAA
C. trachomatis LGV   GAAUGUGGCGAUAUUUGGGCAUCCGAGUAACGUUAAAGAA
C. trachomatis K     GAAUGUGGCGAUAUUUGGGCAUCCGAGUAACGUUAAAGAA
Core Variation              A-auGuuuAggcaucUAaAACaUA
Probe 781             TACACCGCTATAAACCCGTAGGCTCATTGCAATTTC-5'


Pos. # (E. coli)      453                               485
                       |                                 |
Escherichia coli     AAGGGAGUAAAGUUAAUACCUUUGCUCAUUGACGUUA
Chlamydia psittaci   AAGAGAGAUUGGCUAAUAUCCAAUCGAUUUGAGCGUA
C. trachomatis LGV   AAGAGAAGGCGGUUAAUACCCGCUGGAUUUGAGCGUA
Core Variation              AGGCggUuaauaCccGCuG
Probe 860             CTCTTCCGCCAATTATGGGCGACCTAAACTCGC-5'
```

9

EP 0 732 408 A2

**TABLE 1 (cont'd): CHLAMYDIA 16S rRNA PROBES AND TARGET SEQUENCES**

```
Pos. # (E. coli)        602                              634
                        |                                |
Escherichia coli     UCAGAUGUGAAAUCCCCGGGCUCAACCUGGGAACUGCAU
Chlamydia psittaci   UUAGAUGUUAAAUCUUGGGGCUCAACCCCAAGCCAGCAU
C. trachomatis LGV   UUAGUUGUCAAAGAUCGGGGCUCAACCCCGAGUCGGCAU
C. trachomatis K     UUAGUUGUCAAAGAUCGGGGCUCAACCCCGAGUCGGCAU
Core Variation                 CaaaGAuCggggcucaaccccGagUcG
Probe 861                 AACAGTTTCTAGCCCCGAGTTGGGGCTCAGCCG-5'


Pos. # (E. coli)        827                    853
                        |                      |
Escherichia coli     ACUUGGAGGUUGUGCCCU-UGAGGCGUGGCUU
Chlamydia psittaci   ACUUGAUGUGGAUAGUCUCAACCCUAUCCGUG
C. trachomatis LGV   ACUUGAUGUGGAUGGUCUCAACCCCAUCCGUG
C. trachomatis K     ACUUNAUGUGGAUGGUCUCAACCCNAUCNGUG
Core Variation                 GgucucaacccC
Probe 879            AACTACACCTACCAGAGTTGGGGTAGGC-5'


Pos. # (E. coli)        995                                              1046
                        |                                                |
Escherichia coli     GACAUCCACGGAA-GUUUUCAGAGAUGAGAAUGUGCCUUCGGGAACCGUGAGACAGG
Chlamydia psittaci   GACAUGUAUUUGACCGCGGCAGAAAUGUCGUUUUC-CGCAAGGACAGAUA-CACAGG
C. trachomatis LGV   GACAUGUAUAUGACCGCGGCAGAAAUGUCGUUUUC-CGCAAGGACAUAUA-CACAGG
Core Variation            GUaUWUGaCCGCGGcagaAaugUcGUuUuC-cGCAAggaCAKAuA-C
Probe 783            GTACATAAACTGGCGCCGTCTTTACAGCAAAAG-GC-5'
Probe 882                           CGTCTTTACAGCAAAAG-GCGTTCCTGTATAT-GTGT-5'
```

# TABLE 2: CHLAMYDIA 23S rRNA PROBES AND TARGET SEQUENCES

```
Pos. # (E. coli)       80                                      113
                       |                                       |
Escherichia coli     UCGGUAAGGUGAUAUGAACCGUUAUAACCGGCGAUUUC
C. trachomatis LGV   CCGGCGAGCUGGUGAUAAGCAAAG-ACCCGGAGGUAUC
Chlamydia psittaci   UCGGCGAGCUGGUAUAAAGCUAUG-ACCCGGAGGUCUC
Core Variation                       GAUaagcAaAg-acccggagguA
Probe 1318               CCGCUCGACCACUAUUCGUUUC-UGGGCCUCCAU-5'


Pos. # (E. coli)       135                          159  164                    188
                       |                            |    |                      |
Escherichia coli     UGUGUUUCGACACACUAUCAUUA---ACUGAAUCCAUAGGUUAAUGAGGCGA-ACCGGGGGA
C. trachomatis LGV   GGUAGAGUAAUAGACUACCAUUGCAUGCUGAAUACAUAGGUAUGCAAAGCGACACCUGCCGA
Chlamydia psittaci   GGUAGAUUAAUCAUCUACCAUUAUACGUUGAAUACAUAGG-CGUAUAAGGCACNCCUGUUGA
Core Variation             GuaauAGAcuaggauuGCaUgC
Probe 1319             AUCUCAUUAUCUGAUGGUAACGUACGACUUAUG-5'
Core Variation                                              UAUGCAaagCGacaccugCC
Probe 1320                              CUUAUGUAUCCAUACGUUUCGCUGUGGACGG-5'


Pos. # (E. coli)       258                          269       269                            297
                       |                            |         |                              |
Escherichia coli     GGGGAGCAGCCC-----------------------------------AGAGCCUGAAUCAGUGUGUGUGUUAGUGGA
C. trachomatis LGV   GGGGAAUAGCCUAAACCGAGCUGAUAAGGCUCGGGGUUGUAGGAUUGAGGAUAAAGGAUCAGGACUCCUAGUUGA
Chlamydia psittaci   GGGGAAAAGCCUAAACC-ACAUUUUUAAUGU-GGGGUUGUAGGGUCGAUAACAUGGGAUCUUAAGUUUUAGUUGA
Core Variation             UagccuaaaccGaGCuGAuAaGGCuC
Probe 1321           CCCCUUAUCGGAUUUGGCUCGACUAUUCCGAGC-5'
Core Variation                                              AuUgaGGaUaAAggaucAGGaCuCC
Probe 1322                            UCCUAACUCCUAUUUCCUAGUCCUGAGGAUCAA-5'
```

TABLE 2 (cont'd): CHLAMYDIA 23S rRNA PROBES AND TARGET SEQUENCES

```
Pos. # (E. coli)        343                              371
                         |                                |
Escherichia coli     CACAAAAAUGCACAUGCUGUGAGCUCGAU----GAGU
C. trachomatis LGV   GACGAAAGGAGAGAAAGACCGACCUCAACACCUGAGU
Chlamydia psittaci   GACGAAAAAAC-AAGAGACUCUAUUCGAUACCUGAGU
Core Variation                 GGAGAGaAagacCGACCucAaC
Probe 1323               GCUUUCCUCUCUUUCUGGCUGGAGUUGUGGACU-5'


Pos. # (E. coli)        1158                             1193
                         |                                |
Escherichia coli     AGCUGCGGCAGCGACGCUUAUGCGUUGUUGGGUAGGGGAG
C. trachomatis LGV   CAUCGCGGGUGUGUCGAUAAGA---CACGCGGUAGGAGAG
Chlamydia psittaci   AAUCGCGGGUGUAUAUUUAUAU---AUCGCGGUAGGAGAG
Core Variation                 GuCGAuaAGA---CA
Probe 1479               AGCGCCCACACAGCTATTCT---GTGCGCCATCCTC-5'


Pos. # (E. coli)        1457                             1489
                         |                                |
Escherichia coli     GGUUGUCCCGGUUUUAAGCGUGUAGGCUGGGUUUUCC---AGG
C. trachomatis LGV   CGAUUGGAAGAGUCCGUAGAGCGAUGAGAACGGUUAGUAGG
Chlamydia psittaci   CGAUUGGAAAUGUCCGUAUCACAAUGAGACCGGUUAGUAGG
Core Variation              GAguccguaGAGcGaugagaA
Probe 1325               UAACCUUCUCAGGCAUCUCGCUACUCUUGCCAA-5'


Pos. # (E. coli)        1503                             1531
                         |                                |
Escherichia coli     CAAAUCCGGAAAAUCAAGGCUGAGGCGUGAUGACGAGG----CAC
C. trachomatis LGV   CAAAUCCGCUAACAUAAGAUCAGGUCGCGAUCAAGGGGAAUCUUC
Chlamydia psittaci   CAAAUCCGCUAACAUAAGGUUAGGUUGUGGUUAAGGGAAAUCUUC
Core Variation                    AuCagguCgCgAuCaagggG
Probe 1324                  UUGUAUUCUAGUCCAGCGCUAGUUCCCCUUAGA-5'


Pos. # (E. coli)        1708                             1746
                         |                                |
Escherichia coli     CGCUGAUAUGUAGGUGAGGUCCCUCGCGGAUGGAGCUGAAAUC
C. trachomatis LGV   AGCCUU--UUAGGGUGACUAUGGA-ACGAUAGGAGCCCCGGGG
Chlamydia psittaci   AGCCUC--UUAGGGUGAUUgcCUUUACGGCAUGAGCUCCGGGG
Core Variation           U--uuagggugaCuAUGGA-acgAUaGgagcC
Probe 1220               GGAA--AAUCCCACUGAUACCU-UGCUAUCCUCGGGGCC-5'
```

TABLE 3a

| LIQUID HYBRIDIZATION INCLUSIVITY DATA FOR 16S rRNA-TARGETED PROBES | | | | | | | |
|---|---|---|---|---|---|---|---|
| Genus species | strain | PROBE HYBRIDIZATION | | | | | |
| | | 1153 | 1203 | 781 | 860 | 861 | 879 |
| Chlamydia trachomatis | LGV-1 | - | ++++ | ++++ | ++++ | +++ | ++++ |
| Chlamydia trachomatis | LGV-2 | - | ++++ | ++++ | ++++ | +++ | ++++ |
| Chlamydia trachomatis | LGV-3 | - | ++++ | ++++ | ++++ | ++++ | ++++ |
| Chlamydia trachomatis | A | - | - | ++++ | - | ++++ | ++++ |
| Chlamydia trachomatis | B | - | - | ++++ | + | ++++ | +++ |
| Chlamydia trachomatis | Ba | + | - | ++++ | + | +++ | ++++ |
| Chlamydia trachomatis | C | + | - | ++++ | +++ | +++ | +++ |
| Chlamydia trachomatis | D | + | - | ++++ | + | ++++ | ++++ |
| Chlamydia trachomatis | E | - | ++++ | ++++ | + | ++++ | ++++ |
| Chlamydia trachomatis | F | - | ++ | ++++ | - | + | ++ |
| Chlamydia trachomatis | G | + | - | ++++ | + | ++++ | ++++ |
| Chlamydia trachomatis | H | + | - | ++++ | + | ++ | ++ |
| Chlamydia trachomatis | I | + | - | ++++ | + | +++ | ++++ |
| Chlamydia trachomatis | J | + | - | ++++ | + | +++ | ++++ |
| Chlamydia trachomatis | K | ++++ | ++++ | ++++ | ++++ | ++++ | ++++ |
| Bacillus cereus | GT0008 | - | - | - | - | - | - |
| Camphylobacter jejunii | GT0022 | - | - | - | - | - | - |
| Candida albicans | GT0128 | - | - | - | - | - | - |
| Corynebacter genitalium | GT0045 | - | - | - | - | - | - |
| Escherichia coli | GT1720 | - | - | - | - | - | - |
| Neisseria gonorrea | GT0315 | - | - | - | - | - | - |
| "+++++" = positive control level of hybribization, "+" = barely detectable, "-" = zero. | | | | | | | |

Table 3B

| LIQUID HYBRIDIZATION INCLUSIVITY DATA FOR 23S rRNA-TARGETED PROBES | | | | | | | | | | | |
|---|---|---|---|---|---|---|---|---|---|---|---|
| Genus species | strain | PROBE HYBRIDIZATION | | | | | | | | | |
| | | 1318 | 1319 | 1320 | 1321 | 1322 | 1323 | 1324 | 1325 | 1220 |
| Chlamydia trachomatis | LGV-1 | ++++ | ++++ | ++++ | ++++ | ++++ | +++ | ++++ | ++++ | ++++ |
| Chlamydia trachomatis | LGV-2 | ++++ | +++ | ++++ | ++++ | +++ | +++ | ++ | +++ | ++++ |
| Chlamydia trachomatis | LGV-3 | ++++ | ++++ | ++++ | ++++ | +++ | ++++ | +++ | +++ | ++++ |
| Chlamydia trachomatis | A | ++++ | ++++ | ++++ | ++++ | ++ | ++++ | ++ | +++ | ++ |
| Chlamydia trachomatis | B | ++++ | ++++ | ++++ | ++++ | +++ | ++++ | ++ | +++ | ++ |
| Chlamydia trachomatis | Ba | ++++ | ++++ | ++++ | ++++ | +++ | ++++ | ++++ | ++++ | ++++ |
| Chlamydia trachomatis | C | ++++ | ++++ | ++++ | ++++ | ++++ | ++++ | ++++ | ++++ | ++++ |
| Chlamydia trachomatis | D | ++++ | ++++ | ++++ | ++++ | ++++ | ++++ | ++++ | ++++ | ++++ |
| Chlamydia trachomatis | E | ++++ | ++++ | ++++ | ++++ | ++++ | ++ | ++++ | ++++ | ++++ |
| Chlamydia trachomatis | F | ++++ | +++ | ++++ | ++++ | ++++ | + | +++ | +++ | ++ |
| Chlamydia trachomatis | G | ++++ | ++++ | ++++ | ++++ | ++++ | ++++ | ++++ | ++++ | ++++ |
| Chlamydia trachomatis | H | ++++ | ++++ | ++++ | ++++ | ++++ | ++++ | ++++ | ++++ | ++ |
| Chlamydia trachomatis | I | ++++ | ++++ | ++++ | ++++ | ++++ | ++++ | ++++ | ++++ | ++++ |
| Chlamydia trachomatis | J | ++++ | ++++ | ++++ | ++++ | ++++ | ++++ | ++++ | ++++ | ++++ |
| Chlamydia trachomatis | K | ++++ | ++++ | ++++ | ++++ | ++++ | ++++ | ++++ | ++++ | ++++ |
| Bacillus cereus | GT0008 | - | - | - | - | - | - | - | - | - |
| Camphylobacter jejunii | GT0022 | - | - | - | - | - | - | - | - | - |
| Candida albicans | GT0128 | - | - | - | - | - | - | - | - | - |
| Corynebacter genitalium | GT0045 | - | - | - | - | - | - | - | - | - |
| Escherichia coli | GT1720 | - | - | - | - | - | - | - | - | - |
| Neisseria gonorrea | GT0515 | - | - | - | - | - | - | - | - | - |
| "++++" = positive level of hybridization, "+" = barely detectable, "-" = zero. | | | | | | | | | | | |

## TABLE 4A: CHLAMYDIA 16S EXCLUSIVITY DOT-BLOT DATA

PROBE HYBRIDIZATION

| Genus species | strain | 1153 | 1203 | 781 | 860 | 861 | 879 | 783 | 882 |
|---|---|---|---|---|---|---|---|---|---|
| Chlamydia trachomatis (EB) | LGV | + | +++ | ++++ | ++++ | ++++ | ++++ | ++++ | +++ |
| Chlamydia trachomatis (RB) | LGV | - | +++ | ++++ | ++++ | ++++ | ++++ | +++ | ++++ |
| Chlamydia psittaci (EB +RB) | LGV | - | - | - | - | - | - | +++ | +++ |
| Acinetobaterr calcoaceticus | GT0002 | - | - | - | - | - | - | - | - |
| Acinetobacter lwoffii | GT0004 | - | - | - | - | - | - | - | - |
| Aeromonas sobria | GT0007 | - | - | - | - | - | - | - | - |
| Bacillus cereus | GT0008 | - | - | - | - | - | - | - | - |
| Bacteroides fragilis | ATCC29771 | - | - | - | - | - | - | - | - |
| Bacteroides mellanioogenicus | GT0011 | - | - | - | - | - | - | - | - |
| Bacteroides thetaiotamicron | GT0527 | - | - | - | - | - | - | - | - |
| Bifidobacterium dentium | GT0012 | - | - | - | - | - | - | - | - |
| Camphylobacter jejunii | GT0022 | - | - | - | - | - | - | - | - |
| Candida albicans | 223-87 | - | - | - | - | - | - | - | - |
| Candida albicans | 819-88 | - | - | - | - | - | - | - | - |
| Candida tropicalis | 750 | - | - | - | - | - | - | - | - |
| Candida tropicalis | 224-87 | - | - | - | - | - | - | - | - |
| Candida diversus | GT0030 | - | - | - | - | - | - | - | - |
| Citrobacter freundii | GT0687 | - | - | - | - | - | - | - | - |
| Clostridium perfringens | ATCC13124 | - | - | - | - | - | - | - | - |
| Clostridium gentalium | GT0045 | - | - | - | - | - | - | - | - |
| Enterobacter agglomerans | GT0683 | - | - | - | - | - | - | - | - |
| Enterobacter cloacae | GT0686 | - | - | - | - | - | - | - | - |
| Enterobacter sakazakii | GT0062 | - | - | - | - | - | - | - | - |
| Escherichia coli | 1665 | - | - | - | - | - | - | - | - |
| Escherichia hermanii | GT0232 | - | - | - | - | - | - | - | - |
| Escherichia vulneris | GT0233 | - | - | - | - | - | - | - | - |
| Fusobacterium necrophorum | GT0238 | - | - | - | - | - | - | - | - |

EP 0 732 408 A2

TABLE 4A (cont'd): CHLAMYDIA 16S EXCLUSIVITY DOT-BLOT DATA

PROBE HYBRIDIZATION

| Genus species | strain | 1153 | 1203 | 781 | 860 | 861 | 879 | 783 | 882 |
|---|---|---|---|---|---|---|---|---|---|
| Fusobacterium prausnitzii | ATCC27768 | - | - | - | - | - | - | - | - |
| Flavobacterium meningosepticum | GT0037 | - | - | - | - | - | - | - | - |
| Giardia : CDC ( human ) | | - | - | + | - | + | - | - | - |
| Hafnia alvei | GT0241 | - | - | - | - | - | - | - | - |
| Haemophilus influenzae | GT0244 | - | - | - | - | - | - | - | - |
| Kingella dentrificans | GT0245 | - | - | - | - | - | - | - | - |
| Kingella indologenes | GT0246 | - | - | - | - | - | - | - | - |
| Kingella kingae | GT0247 | - | - | - | - | - | - | - | - |
| klebsiella pneumonia | 1500 | - | - | - | - | - | - | - | - |
| Lactobacillus acidophilus | GT0256 | - | - | - | - | - | - | - | - |
| Kingella kingae | GT0247 | - | - | - | - | - | - | - | - |
| Lactobacillus minitus | GT0257 | - | - | - | - | - | - | - | - |
| Lactobacillus casei | GT0805 | - | - | - | - | - | - | - | - |
| Lactobacillus plantarum | GT0258 | - | - | - | - | - | - | - | - |
| Listeria monocytogenes | IG3299 | - | - | - | - | - | - | - | - |
| Morganella morganii | GT0303 | - | - | - | - | - | - | - | - |
| Moraxella osloensis | GT0301 | - | - | - | + | + | - | - | - |
| Neisseria cinerea | GT0307 | - | - | - | - | - | - | - | - |
| Neisseria flavescens | GT0310 | - | - | - | - | - | - | - | - |
| Neisseria gonorrhoea | ATCC19424 | - | - | - | - | - | - | - | - |
| Neisseria meningitidis | GT0349 | - | - | - | - | - | - | - | - |
| Neisseria mucosa | GT0353 | - | - | - | - | - | - | - | - |
| Peptostreptococcus anaerobius | GT0359 | - | - | - | - | - | - | - | - |
| Plesiomonas shigelliodes | ATCC14029 | - | - | - | - | - | - | - | - |
| Proteus mirabilis | 1496 | - | - | - | - | - | - | - | - |
| Proteus vulgaris | GT0368 | - | - | - | - | - | - | - | - |
| Providencia alcalificiens | GT0371 | - | - | - | - | - | - | - | - |

EP 0 732 408 A2

EP 0 732 408 A2

## TABLE 4A (cont'd): CHLAMYDIA 16S EXCLUSIVITY DOT-BLOT DATA

PROBE HYBRIDIZATION

| Genus species | strain | 1153 | 1203 | 781 | 860 | 861 | 879 | 783 | 882 |
|---|---|---|---|---|---|---|---|---|---|
| Providencia rettgeri | GT0373 | - | - | - | - | - | - | - | - |
| Providencia stuartii | GT0375 | - | - | - | - | - | - | - | - |
| Pseudomonas acidovorans | GT0376 | - | - | - | - | - | - | - | - |
| Pseudomonas aeruginosa | 1908 | - | - | - | - | - | - | - | - |
| Salmonella arizona | GT0799 | - | - | - | - | - | - | - | - |
| Salmonella typhimurium | GT0389 | - | - | - | - | - | - | - | - |
| Serratia marcescens | GT0392 | - | - | - | - | - | - | - | - |
| Shigella boydii C-13 | RF 974 | - | - | - | - | - | - | - | - |
| Shigella dysenteriae | RF 970 | - | - | - | - | - | - | - | - |
| Shigella flexnerii | GT0798 | - | - | - | - | - | - | - | - |
| Shigella sonnei | RF 968 | - | - | - | - | - | - | - | - |
| Staphylococcus aureus | GT0399 | - | - | - | - | - | - | - | - |
| Staphylococcus epidermitis | GT0401 | - | - | - | - | - | - | - | - |
| Streptococcus sanguis | GT0411 | - | - | - | - | - | - | - | - |
| Streptococcus agalactiae | GT0405 | - | - | - | - | - | - | - | - |
| Streptococcus faecalis | GT0406 | - | - | - | - | - | - | - | - |
| Streptococcus faecium | GT0407 | - | - | - | - | - | - | - | - |
| Streptococcus mutans | GT0412 | - | - | - | - | - | - | - | - |
| Streptococcus salivarius | GT0410 | - | - | - | - | - | - | - | - |
| Torulopsis globrata | 2001 | - | - | - | - | - | - | - | - |
| Vibrio parahemolyticus | GT0568 | - | - | - | - | - | - | - | - |
| Xanthomonas maltophilia | GT0417 | - | - | - | - | - | - | - | - |
| Yersinia enterocolitica | GT0419 | - | - | - | - | - | - | - | - |
| L-cell RNA | | - | - | - | + | - | - | - | - |
| Human RNA from Whole Blood | | - | - | - | - | - | - | - | - |
| Human stool | | - | - | - | - | - | - | - | - |

\* Inclusivity and Exclusivity data was collected from overnight exposures.
\*\* Each organism is represented by 100ng of CsTFA purified RNA.

TABLE 4B (cont'd): CHLAMYDIA 23S EXCLUSIVITY DOT-BLOT DATA

PROBE HYBRIDIZATION

| Genus species | strain | 1318 | 1319 | 1320 | 1321 | 1322 | 1323 | 1324 | 1325 | 1479 | 1220 |
|---|---|---|---|---|---|---|---|---|---|---|---|
| Pseudomonas aeruginosa | 1908 | – | – | – | – | – | – | – | – | – | – |
| Salmonella arizona | GT0799 | – | – | – | – | – | – | ++ | – | – | – |
| Salmonella typhimurium | GT0389 | – | – | – | – | – | – | + | – | – | – |
| Serratia marcescens | GT0392 | – | – | – | – | – | – | – | – | – | – |
| Shigella boydii C-13 | RF 974 | – | – | – | – | – | – | + | – | – | – |
| Shigella dysenteriae | RF 970 | – | – | – | – | – | – | ++ | – | – | – |
| Shigella flexnerii | GT0798 | – | – | – | – | – | – | ++ | – | – | – |
| Shigella sonnei | RF 968 | – | – | – | – | – | – | + | – | – | – |
| Staphylococcus aureus | GT0399 | – | – | – | – | – | – | – | – | – | – |
| Staphylococcus epidermitis | GT0401 | – | – | – | – | – | – | – | – | – | – |
| Streptococcus sanguis | GT0411 | – | – | – | – | – | – | – | – | – | – |
| Streptococcus agalactiae | GT0405 | – | – | – | – | – | – | – | – | – | – |
| Streptococcus faecalis | GT0406 | – | – | – | – | – | – | – | – | – | – |
| Streptococcus faecium | GT0407 | – | – | – | – | – | – | – | – | – | – |
| Streptococcus mutans | GT0412 | – | – | – | – | – | – | – | – | – | – |
| Streptococcus salivarius | GT0410 | – | – | – | – | – | – | – | – | – | – |
| Torulopsis globrata | 2001 | – | – | – | – | – | – | – | – | – | – |
| Vibrio parahemolyticus | GT0568 | – | – | – | – | – | – | – | – | – | – |
| Xanthomonas maltophilia | GT0417 | – | – | – | – | – | – | – | – | – | – |
| Yersinia enterocolitica | GT0419 | – | – | – | – | – | – | – | – | – | – |
| McCoy cell RNA | | – | – | – | – | – | – | – | – | – | ND |
| Human RNA from Whole Blood | | – | – | – | – | – | – | – | – | – | – |

\*   Inclusivity and Exclusivity data was collected from overnight exposures.
\*\*   Each organism is represented by 100ng of CsTFA purified RNA.

EP 0 732 408 A2

## TABLE 4B (cont'd): CHLAMYDIA 23S EXCLUSIVITY DOT-BLOT DATA

PROBE HYBRIDIZATION

| Genus species | strain | 1318 | 1319 | 1320 | 1321 | 1322 | 1323 | 1324 | 1325 | 1479 | 1220 |
|---|---|---|---|---|---|---|---|---|---|---|---|
| Giardia : CDC ( human ) | | - | - | - | + | - | - | - | - | - | - |
| Hafnia alvei | GT0241 | - | - | - | - | - | - | ++ | - | - | - |
| Haemophilus influenzae | GT0244 | - | - | - | - | - | - | - | - | - | - |
| Kingella dentrificans | GT0245 | - | - | - | - | - | - | - | - | - | - |
| Kingella indologenes | GT0246 | - | - | - | - | - | - | - | - | - | - |
| Kingella kingae | GT0247 | - | - | - | - | - | - | + | - | - | - |
| klebsiella pneumonia | 1500 | - | - | - | - | - | - | - | - | - | - |
| Lactobacillus acidophilus | GT0256 | - | - | - | - | - | - | - | - | - | - |
| Lactobacillus minitus | GT0257 | - | - | - | - | - | - | - | - | - | - |
| Lactobacillus casei | GT0805 | - | - | - | - | - | - | - | - | - | - |
| Lactobacillus plantarum | GT0258 | - | - | - | - | - | - | - | - | - | - |
| Moraxella osloensis | GT0301 | - | - | - | - | - | - | - | + | - | - |
| Neisseria cinerea | GT0307 | - | - | - | - | - | - | - | - | - | - |
| Neisseria flavescens | GT0310 | - | - | - | - | - | - | - | - | - | - |
| Neisseria gonorrhoea | ATCC19424 | - | - | - | - | - | - | - | - | - | - |
| Neisseria meningitidis | GT0349 | - | - | - | - | - | - | - | - | - | - |
| Neisseria mucosa | GT0353 | - | - | - | - | - | - | - | - | - | - |
| Peptostreptococcus anaerobius | GT0359 | - | - | - | - | - | - | - | - | - | - |
| Plesiomonas shigelliodes | ATCC14029 | - | - | - | - | - | - | - | - | - | - |
| Proteus mirabilis | 1496 | - | - | - | - | - | - | - | - | - | - |
| Proteus vulgaris | GT0368 | - | - | - | - | - | - | - | - | - | - |
| Providencia alcalificiens | GT0371 | - | - | - | - | - | - | - | - | - | - |
| Providencia rettgeri | GT0373 | - | - | - | - | - | - | - | - | - | - |
| Providencia stuartii | GT0375 | - | - | - | - | - | - | - | - | - | - |
| Pseudomonas acidovorans | GT0376 | - | - | - | - | - | - | - | - | - | - |

EP 0 732 408 A2

EP 0 732 408 A2

## TABLE 4B: CHLAMYDIA 23S EXCLUSIVITY DOT-BLOT DATA

PROBE HYBRIDIZATION

| Genus species | strain | 1318 | 1319 | 1320 | 1321 | 1322 | 1323 | 1324 | 1325 | 1479 | 1220 |
|---|---|---|---|---|---|---|---|---|---|---|---|
| Chlamydia trachomatis (LGV) | | ++++ | ++++ | ++++ | ++++ | ++++ | ++++ | ++++ | ++++ | ++++ | ++++ |
| Acinetobaterr calcoaceticus | GT0002 | - | - | - | - | - | - | - | - | - | - |
| Acinetobacter lwoffii | GT0004 | - | - | - | - | - | - | - | - | - | - |
| Aeromonas sobria | GT0007 | - | - | - | - | - | - | - | - | - | - |
| Bacillus cereus | GT0008 | - | - | - | - | - | - | - | - | - | - |
| Bacteroides fragilis | ATCC29771 | - | - | - | - | - | - | - | - | - | - |
| Bacteroides mellanioogenicus | GT0011 | - | - | - | - | - | - | - | - | - | - |
| Bacteroides thetaiotamicron | GT0527 | - | - | - | - | - | - | - | - | - | - |
| Bifidobacterium dentium | GT0012 | - | - | - | - | - | - | - | - | - | - |
| Candida albicans | ATCC60193 | - | - | - | - | - | - | - | - | - | - |
| Candida albicans | ATCC36232 | - | - | - | - | - | - | - | - | - | - |
| Candida tropicalis | 750 | - | - | - | - | - | - | - | - | - | - |
| Candida diversus | GT0030 | - | - | - | - | - | - | ++ | - | - | - |
| Citrobacter freundii | GT0687 | - | - | - | - | - | - | ++ | - | - | - |
| Clostridium perfringens | ATCC13124 | - | - | - | - | - | - | - | - | - | - |
| Clostridium gentalium | GT0045 | - | - | - | - | - | - | - | - | - | - |
| Enterobacter agglomerans | GT0683 | - | - | - | - | - | - | ++ | - | - | - |
| Enterobacter cloacae | GT0686 | - | - | - | - | - | - | + | - | - | - |
| Enterobacter sakazakii | GT0062 | - | - | - | - | - | - | + | - | - | - |
| Escherichia coli | 1665 | - | - | - | - | - | - | + | - | - | - |
| Escherichia hermanii | GT0232 | - | - | - | - | - | - | +++ | - | - | - |
| Escherichia vulneris | GT0233 | - | - | - | - | - | - | +++ | - | - | - |
| Fusobacterium necrophorum | GT0238 | - | - | - | - | - | - | - | - | - | - |
| Fusobacterium prausnitzii | ATCC27768 | - | - | - | - | - | - | - | - | - | - |
| Flavobacterium meningosepticum | GT0037 | - | - | - | - | - | - | - | - | - | - |

SEQUENCE LISTINGS

SEQ ID NO: 1
SEQUENCE LENGTH: 36 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No


SEQUENCE:


CTTTAACGTT ACTCGGATGC CCAAATATCG CCACAT          36



SEQ ID NO: 2
SEQUENCE LENGTH: 35 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No


SEQUENCE:


CTTTAATATG TTTTAGATGC CTAAACATAC CACAT          35

SEQ ID NO: 3

SEQUENCE LENGTH: 35 bases

SEQUENCE TYPE: Nucleotide

STRANDEDNESS: Single

TOPOLOGY: Linear

MOLECULE TYPE: DNA probe for <u>Chlamydia trachomatis</u>

HYPOTHETICAL: No


SEQUENCE:


CGGAAAACGA   CATTTCTGCC   GCGGTCAAAT   ACATG               35


SEQ ID NO: 4

SEQUENCE LENGTH: 33

SEQUENCE TYPE: Nucleotide

STRANDEDNESS: Single

TOPOLOGY: Linear

MOLECULE TYPE: DNA probe for <u>Chlamydia trachomatis</u>

HYPOTHETICAL: No


SEQUENCE:


CGCTCAAATC   CAGCGGGTAT   TAACCGCCTT   CTC               33

SEQ ID NO: 5
SEQUENCE LENGTH: 33 bases
SEQUENCE TYPE: Nucleotide
STRANDEDNESS: Single
TOPOLOGY: Linear
MOLECULE TYPE: DNA probe for <u>Chlamydia trachomatis</u>
HYPOTHETICAL: No


SEQUENCE:


GCCGACTCGG  GGTTGAGCCC  CGATCTTTGA  CAA                    33


SEQ ID NO: 6
SEQUENCE LENGTH: 28 bases
SEQUENCE TYPE: Nucleotide
STRANDEDNESS: Single
TOPOLOGY: Linear
MOLECULE TYPE: DNA probe for <u>Chlamydia trachomatis</u>
HYPOTHETICAL: No


SEQUENCE:


CGGATGGGGT  TGAGACCATC  CACATCAA                    28

SEQ ID NO: 7
SEQUENCE LENGTH: 35 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No


SEQUENCE:


TGTGTATATG  TCCTTGCGGA  AAACGACATT  TCTGC          35


SEQ ID NO: 8
SEQUENCE LENGTH: 35 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No


SEQUENCE:


CCACTAAACA  ATCGTCGAAA  CAATTGCTCC  GTTCG          35

SEQ ID NO: 9
SEQUENCE LENGTH: 35 bases
SEQUENCE TYPE: Nucleotide
STRANDEDNESS: Single
TOPOLOGY: Linear
MOLECULE TYPE: DNA probe for <u>Chlamydia trachomatis</u>
HYPOTHETICAL: No

SEQUENCE:

CCACTAAACA   ATTGCCGAAA   CAATTGCTCC   GTTCG              35

SEQ ID NO: 10
SEQUENCE LENGTH: 35 bases
SEQUENCE TYPE: Nucleotide
STRANDEDNESS: Single
TOPOLOGY: Linear
MOLECULE TYPE: DNA probe for <u>Chlamydia trachomatis</u>
HYPOTHETICAL: No

SEQUENCE:

CCGGGGCTCC   TATCGTTCCA   TAGTCACCCT   AAAAG              35

SEQ ID NO: 11
SEQUENCE LENGTH: 33 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No

SEQUENCE:

TACCTCCGGG  TCTTTGCTTA  TCACCAGCTC  GCC

SEQ ID NO: 12
SEQUENCE LENGTH: 33 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No

SEQUENCE:

GTATTCAGCA  TGCAATGGTA  GTCTATTACT  CTA                    33

SEQ ID NO: 13
SEQUENCE LENGTH: 33 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No


SEQUENCE:


TCGGCAGGTG  TCGCTTTGCA  TACCTATGTA  TTC                33


SEQ ID NO: 14
SEQUENCE LENGTH: 33 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No


SEQUENCE:


CGAGCCTTAT  CAGCTCGGTT  TAGGCTATTC  CCC                33

SEQ ID NO: 15
SEQUENCE LENGTH: 33 bases
SEQUENCE TYPE: Nucleotide
STRANDEDNESS: Single
TOPOLOGY: Linear
MOLECULE TYPE: DNA probe for <u>Chlamydia trachomatis</u>
HYPOTHETICAL: No


SEQUENCE:


AACTAGGAGT  CCTGATCCTT  TATCCTCAAT  CCT                33


SEQ ID NO: 16
SEQUENCE LENGTH: 33 bases
SEQUENCE TYPE: Nucleotide
STRANDEDNESS: Single
TOPOLOGY: Linear
MOLECULE TYPE: DNA probe for <u>Chlamydia trachomatis</u>
HYPOTHETICAL: No


SEQUENCE:


TCAGGTGTTG  AGGTCGGTCT  TTCTCTCCTT  TCG                33

SEQ ID NO: 17
SEQUENCE LENGTH: 33 bases
SEQUENCE TYPE: Nucleotide
STRANDEDNESS: Single
TOPOLOGY: Linear
MOLECULE TYPE: DNA probe for <u>Chlamydia trachomatis</u>
HYPOTHETICAL: No


SEQUENCE:


AGATTCCCCT    TGATCGCGAC    CTGATCTTAT    GTT                    33


SEQ ID NO: 18
SEQUENCE LENGTH: 33 bases
SEQUENCE TYPE: Nucleotide
STRANDEDNESS: Single
TOPOLOGY: Linear
MOLECULE TYPE: DNA probe for <u>Chlamydia trachomatis</u>
HYPOTHETICAL: No


SEQUENCE:


AACCGTTCTC    ATCGCTCTAC    GGACTCTTCC    AAT                    33

```
SEQ ID NO: 19
SEQUENCE LENGTH: 33 bases
SEQUENCE TYPE: Nucleotide
STRANDEDNESS: Single
TOPOLOGY: Linear
MOLECULE TYPE: DNA probe for Chlamydia trachomatis
HYPOTHETICAL: No


SEQUENCE:


CTCCTACCGC  GTGTCTTATC  GACACACCCG  CGA                    33
```

**Claims**

1. A nucleic acid probe complementary or homologous to at least 90% of a nucleotide sequence comprising any ten consecutive nucleotides of the region 453 to 485 or 995 to 1046 of the 16S rRNA of either C. *trachomatis*, or of the region 80 to 113, 135 to 159, 164 to 188 or 258 to 269 of the 23S rRNA of C. *trachomatis* (using the E. *coli* numbering system).

2. A probe as claimed in claim 1 which is probe 860 having the sequence:
   5'-CGCTCAAATCCAGCGGGTATTAACCGCCTTCTC-3';
   probe 783 having the sequence:
   5'-CGGAAAACGACATTTCTGCCGCGGTCAAATACATG-3';
   probe 882 having the sequence:
   5'-TGTGTATATGTCCTTGCGGAAAACGACATTTCTGC-3';
   probe 1318 having the sequence:
   5'-TACCTCCGGGTCTTTGCTTATCACCACTCGCC-3';
   probe 1319 having the sequence:
   5'-GTATTCAGCATGCAATGGTAGTCTATTACTCTA-3';
   probe 1320 having the sequence:
   5'-TCGGCAGGTGTCGCTTTGCATACCTATGTATTC-3';
   probe 1321 having the sequence:
   5'-CGAGCCTTATCAGCTCGGTTTAGGCTATTCCCC-3';
   or one of their complementary sequences.

3. A probe as claimed in claim 2 which is probe 860 having the sequence:
   5'-CGCTCAAATCCAGCGGGTATTAACCGCCTTCTC-3'.

4. A probe as claimed in claim 2 which is probe 783 having the sequence:
   5'-CGGAAAACGACATTTCTGCCGCGGTCAAATACATG-3'.

5. A probe as claimed in claim 2 which is probe 882 having the sequence:
   5'-TGTGTATATGTCCTTGCGGAAAACGACATTTCTGC-3'.

6. A probe as claimed in claim 2 which is probe 1318 having the sequence:
   5'-TACCTCCGGGTCTTTGCTTATCACCACTCGCC-3'.

7. A probe as claimed in claim 2 which is probe 1319 having the sequence:
5'-GTATTCAGCATGCAATGGTAGTCTATTACTCTA-3'.

8. A probe as claimed in claim 2 which is probe 1320 having the sequence:
5'-TCGGCAGGTGTCGCTTTGCATACCTATGTATTC-3'.

9. A probe as claimed in claim 2 which is probe 1321 having the sequence:
5'-CGAGCCTTATCAGCTCGGTTTAGGCTATTCCCC-3'.

10. A method of detecting the presence of *C. trachomatis* in a sample comprising:

(a) contacting the sample with at least one probe as claimed in claim 1; and

(b) detecting a hybrid nucleic acid complex as an indication of the presence of *C. trachomatis* in the sample.

11. A method as claimed in claim 10 wherein the probe is as defined in any one of claims 3 to 9.